# EUROPEAN PATENT APPLICATION

(11) **EP 3 761 193 A1**
(43) Date of publication of application: **06.01.2021**
(21) Application number: 19184391.1
(22) Date of filing: 04.07.2019
(51) Int. Cl.: G06F 17/50, H04L 29/06, H04W 12/00, H04W 4/90, H04L 29/08, G06N 3/00, G05B 19/418

(54) **SAFETY ANALYSIS OF TECHNICAL SYSTEMS COMPRISING HUMAN OBJECTS**

(71) Applicant: Siemens Aktiengesellschaft, 80333 München (DE)
(72) Inventor: Kaukewitsch, Christof, 81479 München (DE); Heilmann, Reiner, 85658 Egmating (DE); Zeller, Marc, 81243 München (DE)

(57) **Abstract**

A computer-implemented method (10) is provided for safety analysis of a technical system comprising a human object. The method comprises: determining (101) a system model (20) of the technical system comprising the human object; determining (102) at least one use case (201) of the technical system in accordance with a human interaction of the human object with the technical system; and simulating (103) the technical system in accordance with the system model (20) and the at least one use case (201). The simulating (103) of the technical system comprises tracking (1034) of safety hazard events in relation to the human interaction.

## Description

### FIELD OF THE INVENTION

Various embodiments of the invention relate to safety analysis of a technical system which comprises a human object. The technical system of interest may particularly relate to a system of systems, a cyber-physical system, an autonomous system, or an artificial intelligence, AI, based system.

### BACKGROUND OF THE INVENTION

Safety, reliability or availability artifacts are essential with respect to the homologation (i.e., authorization procedure) of complex systems or solutions as well as with respect to the fulfillment of corresponding contractual obligations in general.

Presently, safety hazard identification and risk analyses, which include Safety Integrity Level, SIL, Automotive Safety Integrity Level, ASIL, Development Assurance Level, DAL, or Performance Level, PL, assignments as well as Failure Modes and Effects (and Criticality) Analysis, FME(C)A, involve a great deal of manual activities for safety experts and/or other experts, typically being valuable human resources such as system architects, developers or assessors. Significant efforts, expenses and time for experts are involved especially in case of complicated or challenging applications.

In case of adaptive or autonomous systems new potential system compositions have to be judged upfront. Totally new system combinations as for instance to be expected in the context of cyber-physical systems, in which physical and software components are deeply intertwined, may lack a safety risk evaluation and hence cannot be trusted unless a new evaluation by experts has been conducted.

However, today's purely manual safety hazard identification and analysis process fails to enable an a-priori analysis of all possible safety hazards in an autonomous or cyber-physical system. This is a significant limitation for the runtime operation of cyber-physical systems as well as other systems.

### BRIEF SUMMARY OF THE INVENTION

In view of the above, there is a continued need in the art for addressing some of the above needs.

These underlying objects of the invention are each solved by the features of a method as defined by the independent claim. Preferred embodiments of the invention are set forth in the dependent claims.

The invention enables or facilitates the identification, evaluation or validation of safety-relevant hazards or safety requirements by means of a model-based approach with model objects reflecting human behavior, human interaction and relevant properties such as human resilience or sensitivity with regard to specific loads or exposures.

According to a first aspect, a computer-implemented method for safety analysis of a technical system comprising a human object is provided. The method comprises: determining a system model of the technical system comprising the human object; determining at least one use case of the technical system in accordance with a human interaction of the human object with the technical system; and simulating the technical system in accordance with the system model and the at least one use case. The simulating of the technical system comprises tracking of safety hazard events in relation to the human interaction.

The system model of the technical system may comprise at least one human model; and the at least one human model may comprise a model of the human interaction.

The human model may further comprise at least one of: a property model, a behavior model, and a resilience model of a human body.

The resilience model may comprise at least one of: a whole-body damage threshold, and limb-resolved damage thresholds. The tracking of the safety hazard events in relation to the human interaction may comprise identifying that the at least one human model indicates human injury based on the at least one threshold.

The resilience model of the human body may comprise time-resolved damage thresholds and damage accumulation rulesets.

The simulating of the technical system may comprise establishing classes of equivalent safety hazard events with respect to the human interaction, and omitting multiple simulation runs for equivalent safety hazard events.

The establishing of the classes of equivalent safety hazard events may comprise using a method of artificial intelligence, AI.

The tracking of the safety hazard events in relation to the human interaction may comprise at least one of: tracking a respective human injury, tracking a respective human exposure time to, and tracking a respective human avoidance behavior in relation to the tracked safety hazard events.

The method may further comprise evaluating the tracked safety hazard events by evaluating a risk in accordance with at least one of: a Safety Integrity Level, SIL, evaluation model, an Automotive Safety Integrity Level, ASIL, evaluation model, a Development Assurance Level, DAL, evaluation model, a Performance Level, PL, evaluation model, and an individual evaluation model.

The evaluating of the tracked safety hazard events may comprise at least one of: statistically evaluating at least one of: the tracked human injuries, the tracked human exposure times, and the tracked human avoidance behavior; and checking the tracked safety hazard events against a list of known safety hazard events.

The system model of the technical system may further comprise at least one failure mode of the technical system of: a deviation from a design or operating specification of the technical system, a random failure of a component of the technical system, a random human interaction with the component of the technical system; and a systematic human interaction with the component of the technical system. The simulating of the technical system comprises establishing the at least one failure mode.

The establishing of the at least one failure mode may comprise establishing at most one of the at least one failure mode at a time.

The system model of the technical system may further comprise an environmental model including at least one of: a geographical model, a climatic model, a political model, and a jurisdictional model.

The system model of the technical system may further comprise a domain model including at least one of: a hazard-related property, and a hazard-related rule.

The system model of the technical system may further comprise a state model of the technical system in accordance with the at least one use case including a plurality of operational states and at least one transition relation between the plurality of operational states. The simulating of the technical system comprises simulating the technical system in at least one of the plurality of operational states of the state model.

According to a second aspect, a device for safety analysis of a technical system comprising a human object is provided. The device comprises: a first determining unit for determining a system model of the technical system comprising the human object; a second determining unit for determining at least one use case of the technical system in accordance with a human interaction of the human object with the technical system; and a simulation unit for simulating the technical system in accordance with the system model and the at least one use case. The simulating of the technical system comprises tracking of safety hazard events in relation to the human interaction.

According to a third aspect, a computer program or a computer program product or a computer-readable storage medium includes program code that can be executed by at least one processor. Executing the program code causes the at least one processor to perform a method for safety analysis of a technical system comprising a human object. The method comprises: determining a system model of the technical system comprising the human object; determining at least one use case of the technical system in accordance with a human interaction of the human object with the technical system; and simulating the technical system in accordance with the system model and the at least one use case.

### BRIEF DESCRIPTION OF THE DRAWINGS

Embodiments of the invention will be described with reference to the accompanying drawings, in which the same or similar reference numerals designate the same or similar elements.
- Fig. 1: illustrates a computer-implemented method according to an embodiment for safety analysis of a technical system comprising a human object.
- Fig. 2: illustrates a system model of the technical system according to an embodiment.

### DETAILED DESCRIPTION OF EMBODIMENTS

Exemplary embodiments of the invention will now be described with reference to the drawings. While some embodiments will be described in the context of specific fields of application, the embodiments are not limited to this field of application. Further, the features of the various embodiments may be combined with each other unless specifically stated otherwise.

The drawings are to be regarded as being schematic representations and elements illustrated in the drawings are not necessarily shown to scale. Rather, the various elements are represented such that their function and general purpose become apparent to a person skilled in the art.

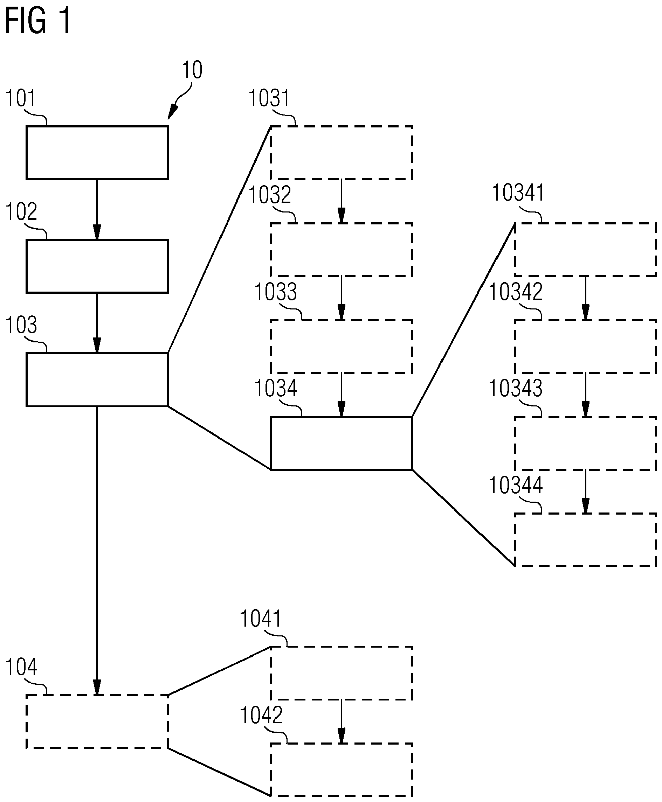

Fig. 1 illustrates a computer-implemented method 10 according to an embodiment for safety analysis of a technical system comprising a human object.

"Safety" as used herein may relate to human safety, and particularly refer to a condition of being safe from undergoing injury, or loss (as a casualty, or fatality).

"Hazard" or "safety hazard" as used herein may refer to a potential source of danger for human safety. Likewise, "safety analysis" as used herein may particularly refer to analysis of human safety.

"Technical system" as used herein may refer to a technical entity (e.g., plant, network, cyber-physical system, System of Systems, autonomous system, adaptive systems) comprising interacting technical (e.g., machines, components, parts) and/or human objects, i.e., human system entities/components/sub-systems. The technical entity may be large and/or complex. Thus, in the technical system, technical components may interact with human objects.

"Cyber-physical systems", CPS, may refer to engineered systems that are built from, and depend upon, a seamless integration of computation and physical components. More specifically, CPS integrate sensing, computation, control and networking into physical objects and infrastructure, connecting them to the Internet and to each other, and enable levels of adaptability, autonomy and functionality that will far exceed the simple embedded systems of today. For example, CPS may comprise driverless cars that communicate securely with each other to reduce delays. As another example, CPS may include smart grids using sensors to analyze the environment and optimize lighting, heating and cooling.

"Human interaction" as used herein may refer to one or more human objects actively and/or passively interacting with other system components, such as technical system components and/or human objects of the technical system. For example, a human interaction may comprise opening a door, escaping upon an emerging stampede, or being present in the technical system, such as sleeping in presence of a - potentially failure-prone - smoke detector. The human interaction may be uni-directional, i.e., the human object affecting one or more further technical components or vice versa; or bidirectional.

"Human object" as used herein may refer to a component of the technical system that has a behavior that is fully or significantly affected by the behavior of a human.

As illustrated, the method comprises steps 101, 102, 103 and sub-step 1034.

In step 101, a system model 20 of the technical system is determined.

The technical system may comprise one of: a system of systems, a cyber-physical system, an autonomous system, and an artificial intelligence, AI, based system.

This may involve a full physical and functional description/modeling of the technical system of interest. The overall system model 20 may comprise models of entities/components/sub-systems to be combined according to given system requirements.

The system entities/components/sub-systems may be modeled as having given properties (e.g. they may be inflammable, poisonous, explosive with a certain "danger" radius etc.), functions, functional relations and failure modes 203 with e.g. certain failure rates and/or corresponding down times and/or corresponding repair times.

Modeling may resort to more or less complex models, such as a two-dimensional model involving certain limitations, or a three-dimensional model.

Modeling may involve the usage of various discrete or continuous distributions in order to account for potential differences of the involved system entities/components/subsystems. For example, human models 202 may be small-tall, old-young, fast-slow etc. Concrete implementations of these distributed values are generated by means of random number generators.

Rule-based modeling may be applied if knowledge about typical hazardous scenarios or design patterns is available. For example, exposing human object models to massive objects with a certain relative speed will result in severe hazards even without knowing which injuries or fatalities will arise in detail. Similar consideration apply for an open door at a relevant train speed, e.g., at more than 40 km/h. This enables simplifying the modeling and speeding up simulations in known environments.

As further detailed below, the system model 20 may also include descriptions of the failure modes 203 of the system entities/components/sub-systems, and may further comprise at least one human model 202, an environmental model 204, and a domain model 205.

With regard to modeling human behavior or human-human interaction by the at least one human model 202, reference may be made to game theory.

In general, it is assumed that manufacturers or suppliers of technical entities/components/sub-systems provide corresponding standardized descriptions ("digital twin", e.g. a semiformal description in a modeling language such as Systems Modeling Language, SysML®) which progressively facilitates seamless composition of system models 20 of complex technical systems based on the standardized descriptions/models of the underlying system entities/components/sub-systems.

All these models are combined to one overall system model 20 to be investigated and evaluated by subsequent simulation runs. System models 20 may for instance be composed of CAD/CAM models, MATLAB/Simulink, UML/SysML models, etc.

System model configurations or realizations shall only be allowed given that specific hazard-related features are not violated. Such may, for instance, comprise easily inflammable material, sharp edges or an accessible high voltage source in a train compartment.

In step 102, at least one use case 201 of the technical system is determined in accordance with a human interaction of the human object with the technical system.

In general, a "use case" may refer to all interactions between a role/actor and a system to achieve a goal. As used herein, a "use case" may refer to all human interactions with the technical system of interest to achieve a goal. For example, a use case of "riding a train" may include human interactions such as "opening a door of a train car", "entering a train compartment", etc.

The at least one use case 201 delimits the usage scenario(s) anticipated for the technical system.

In step 103, the technical system is simulated in accordance with the system model 20 and the at least one use case 201.

The simulating 103 of the technical system may be performed in accordance with at least one configuration parameter.

For example, the simulating 103 may involve e.g. 10.000 simulation runs using differing, randomly sampled number of passengers following a Monte Carlo approach. Random human behavior may be established in these simulation runs. The randomly selected scenarios may be run though a plurality of operational states as further detailed below, and following the at least one use case 201 mentioned above.

As a further example, the simulating 103 may involve performing simulation runs according to a convergence criterion, such as a certain number of simulative runs without yielding new evidence (no additional hazards, no unfavorable statistical trend) or results in a certain convergence interval. Thus, the simulation runs may follow certain strategies, i.e., according to a learning curve.

The method 10 enables or facilitates fast (semi-)automated identification of safety-relevant hazards, such as by identifying a severe (e.g. run number 2987) and a minor (run number 7432) safety hazard to the at least one human object model, for example. The method has a high relevance for complex systems, such as system of systems, cyber-physical systems, autonomous systems, artificial intelligence, AI, based systems etc., and products with safety relevance, especially if functional safety is relevant.

The method 10 enables or facilitates fast (semi-)automated evaluation of safety-relevant hazards, such as by generating SIL, ASIL, DAL or PL assignments for system functions much faster, more comprehensively, more reliably, more precisely, and with a higher level of consistency. This results in a higher quality of hazard/safety risk analyses, shorter time to market and reduced cost for the development of corresponding complex system such as railway products, power or production plants, power distribution etc. In particular, the method may even be fundamentally required for evaluation of adaptive or autonomous systems with runtime integration and/or changing configurations, since it allows for fast and automated reaction to a changed system or system environment, and thus may be useful for outpacing the general technological progress.

The method 10 further enables or facilitates fast (semi-)automated validation of safety-relevant hazards, such as by verifying or validating existing safety hazards or SIL, ASIL, DAL or PL assignments, and thus increasing the confidence in existing hazard and risk analyses.

The method 10 further enables or facilitates detection of design weaknesses even without failure modes of the system entities/components/sub-systems being present. For example, by taking human behavior of bikers and pedestrians into account it will be detected that a pedestrian/cycle track crossing a railway track will frequently involve accidents without making provisions for safety related measures, even without system failure modes.

The method 10 further enables or facilitates deriving additional information with regard to reliability, availability and maintainability of the technical system of interest.

The method 10 further enables or facilitates a high degree of objectivity, since less human judgment is involved to identify hazards and to conduct risk evaluations (including SIL, ASIL, DAL or PL assignments) given adequately validated models.

The method 10 further enables or facilitates the above-identified analyses in ever more complex scenarios, which may denote an intellectual challenge.

In summary, the method 10 may assist, complement or even replace conventional, manual hazard identification and risk analyses by generating comprehensive and reliable results faster than today by means of model-based (semi-) automated processing. In particular, the method 10 is not focused on a specific application area.

In sub-step 1034 of step 103, the simulating of the technical system comprises tracking of safety hazard events in relation to the human interaction.

"Tracking" as used herein may refer to identifying certain events among all emerging events, and maintaining the identified events for subsequent processing, e.g., by buffering.

The computer-implemented method 10 according to the above-described embodiment may be performed by a device having corresponding features: a first determining unit for determining 101 the system model 20 of the technical system comprising the human object; a second determining unit for determining 102 at least one use case 201 of the technical system in accordance with a human interaction of the human object with the technical system; and a simulation unit for simulating 103 the technical system in accordance with the system model 20 and the at least one use case 201. The simulating 103 of the technical system comprises the sub-step 1034 of tracking of safety hazard events in relation to the human interaction.

Advantageously, the technical effects and advantages described above in relation with the method 10 equally apply to the corresponding device.

As illustrated, the step 103 of simulating the technical system may comprise sub-steps 1031, 1032 of establishing 1031 classes of equivalent safety hazard events with respect to the human interaction, and omitting 1032 multiple simulation runs for equivalent safety hazard events.

"Equivalent" as used herein may refer to corresponding (or virtually identical) in effect.

The method 10 enables or facilitates reducing the number of simulation runs of the system model of the technical system in accordance with the at least one use case if certain scenarios are identified that amount to the same result, i.e., lead to the same critical hazard.

Thus, if results can already be anticipated, the corresponding simulation runs may be avoided. An example for this would be to identify a severe hazard in case a passenger falls out of the train at higher speed. As a consequence for even higher train speeds the same result can be expected given the same circumstances. In this sense equivalent hazard classes may be defined to reduce simulation effort.

The step 1031 of establishing the classes of equivalent safety hazard events may comprise using a method of artificial intelligence, AI.

"Artificial intelligence" as used herein may refer to computer-implemented methods that are capable of performing specific tasks effectively without using explicit instructions, only relying on patterns and inference instead. Based on sample/training data, a mathematical model/function is established/learnt which may make predictions or decisions without being explicitly programmed to perform the task. For example, supervised learning establishes/learns the mathematical model/function based on sample/training data that comprises both input data and corresponding desired output data. Once the model/function is established/learnt, it may be used to predict the output data associated with new input data that was not part of the sample/training data.

For example, artificial neural networks may be used as the model/function. The edges of artificial neural networks typically have associated weights which are adjusted as learning proceeds.

In particular, artificial neural networks may be used to identify classes of equivalent or similar safety hazards and to perform classification of safety hazard events accordingly, to increase efficiency of the simulation approach.

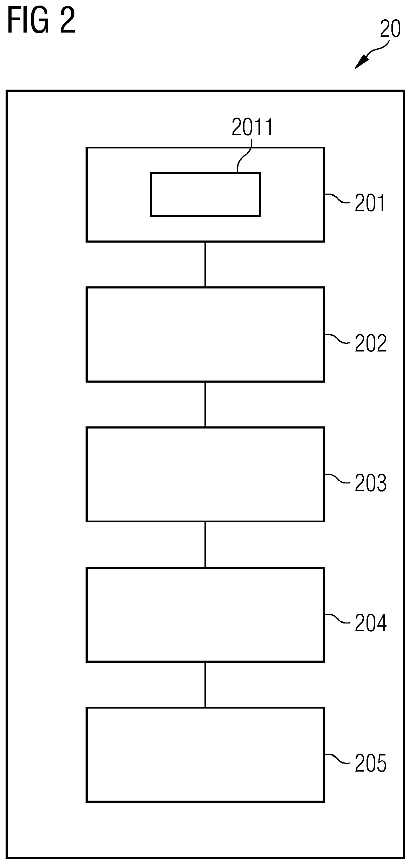

As illustrated, the step 103 of simulating the technical system may comprise a sub-step 1033 of establishing at least one failure mode 203 (which is further defined below with reference to Fig. 2) of the technical system.

"Failure mode" as used herein may refer to a condition or state in which a safety hazard may emerge. For example, a system entity/component/sub-system deviating from the given design or operating specifications of the technical system may or may not result in a safety hazard. As another example, the mere proximity of properly working system entity/component/sub-systems, such as a balcony railing and a human having a body height that is well above average, may or may not result in a safety hazard especially when the human leans on the railing. This latter example illustrates that a failure mode of the at least one failure mode may also be established by mere assembly of the system model, and that any corresponding safety hazard may become evident by the step 103 of simulating the technical system.

Randomly occurring failure modes 203 may be injected to the simulation runs to determine and/or investigate potential hazards. Enabling more than one failure mode at a time may lead to failure combinations. This could e.g. comprise all train brakes braking simultaneously with maximum torque each.

In case of the railroad crossing example above all failure modes may be activated randomly. This includes failures of warning signals and gates which randomly occur simultaneously and potentially resulting in a relevant safety hazard. It may also be possible to include known predefined failure scenarios; e.g. according to domain model data. A resulting model could for instance comprise: a train compartment loading passengers at a train station and then transporting them at high speed through a depopulated mountainous area to the next train station. Only a small number of passengers are sitting on booked seats. There are no sharp edges or explosives in the compartment. The compartment doors comprise the function to open and to close and hence allowing human model objects to enter or to leave the compartment. The door function has certain failures mode leading to opening the doors when not intended, e.g. at high speed, or to not opening the door when required, as for instance in the terminal station or in case of fire in the compartment during a halt.

The step 1033 of establishing the at least one failure mode 203 may comprise establishing at most one of the at least one failure mode 203 at a time.

"Establishing" as used herein may relate to "causing", "effecting" or "bringing about".

The method 10 further enables or facilitates (semi-)automatic generation of FME(C)A analyses by means of simulation, if only one failure mode at a time is enabled which failure mode relates to a deviation from the given design or operating specifications of the technical system, since FME(C)A assesses the failure modes and their resulting effects on the system for each functional or structural system entity/component/sub-system individually. By contrast, failure modes not relating to individual system entities/components/sub-systems, such as failure modes established by mere assembly of the system model, are not of interest for FME(C)A.

If available the occurrence value may be derived from failure rate data or probability of failure of components or sub systems. Severity with regard to hazardous events may be determined in the course of the simulation.

An example for this again could be a more sophisticated railroad crossing with warning signals and railroad crossing gates. In case the warning signal (1 failure) fails to warn pedestrians/bikers the closed gate will prevent safety hazards to bikers or pedestrians from emerging. In case the gate fails to close the warning signals should still prevent safety hazards from emergence. However in case the signalization for the warning signals and the gates is erroneous (1 failure, warning signals and gates remains open) a relevant safety hazard will occur.

As illustrated, the step 1034 of tracking the safety hazard events in relation to the human interaction may comprise at least one of: a sub-step 10341 of identifying that at least one human model 202 of the technical system 20 indicates human injury based on at least one threshold of a resilience model of a human body; a sub-step 10342 of tracking a respective human injury; and sub-steps 10343, 10344 of tracking a respective human exposure time to and tracking a respective human avoidance behavior in relation to the tracked safety hazard events.

"Human injury" as used herein may refer to a compromise/breach of a human's health and/or physical integrity, as far as a human casualty, for example.

"Exposure time" as used herein may refer to a time duration of an exposure to a potentially hazardous situation, i.e., a potential safety hazard.

"Avoidance behavio(u)r" as used herein may refer to a potential human response to an exposure to a safety hazard. Depending upon a response time defined by the respective safety hazard, an avoidance behavior may or may not be shown. For example, a turbine blade detaching from a turbine in operation may be too dynamic for humans to respond, when exposed to this safety hazard.

In particular, it may be monitored and logged during simulation whether humans can escape from a potential threat. This is only possible if perception and reaction to a potential threat is successful, which typically depend on proximity to the hazardous source and is determined by the dynamics of the corresponding event.

For example, a train compartment door opens at high speed. Human model objects that randomly move (behavior model) in the compartment exit the compartment and fall out of the train resulting in severe consequences such as casualties or injuries due to the detected high relative speed between the corresponding human model objects and environmental model objects.

As another example, the train enters a station. The door opens and passengers leave the train. Then suddenly the train door closes and a passenger is clamped between the doors. Since the resulting forces on the human are below the threshold for injury this is not judged as a hazardous event.

As a further example, a high voltage source is placed in the train compartment in a dedicated cabinet. Access to this cabinet is only possible for the maintenance team which possesses a dedicated key for a locked door. During simulation with passengers only in the compartment a hazard with regard to this high voltage source cannot be identified. The reason is that the behavior model for the human passenger does not allow access to this cabinet. This may certainly be different if maintenance staff is present.

As illustrated, the method 10 may further comprise a step 104 in which the tracked safety hazard events are evaluated by evaluating a risk in accordance with at least one of: a Safety Integrity Level, SIL, evaluation model, an Automotive Safety Integrity Level, ASIL, evaluation model, a Development Assurance Level, DAL, evaluation model, a Performance Level, PL, evaluation model, and an individual evaluation model.

For example, risk evaluation may be performed according to IEC 61508 Safety Integrity Level, SIL, or EN ISO 13849-1 Performance Level, PL, or according to individual evaluation models which may also be based on simulation results, e.g., on the basis to predefined acceptable risks or statistical target values.

Alternative domain models for the automotive and avionics software systems include ISO26262 Automotive Safety Integrity Level, ASIL, and DO-178C Development Assurance Level, DAL, respectively.

For example, the simulation results with regard to hazard consequences, exposure time, avoidance of hazard and frequency/probability of unwanted occurrence may be used to determine that an electrical/electronic/programmable electronic, E/E/PS, safety-related function is required with a certain SIL according to IEC 61508-5.

As illustrated, the step 104 of evaluating the tracked safety hazard events may comprise at least one of: a sub-step 1041 of statistically evaluating at least one of: the tracked human injuries, the tracked human exposure times, and the tracked human avoidance behavior; and a sub-step 1042 of checking the tracked safety hazard events against a list of known safety hazard events.

Statistical relevance may be derived by means of a Monte Carlo Simulation. The simulation runs are statistically evaluated (sub-step 1041).

In particular, safety hazard detection and evaluation during simulation may be performed by checking in all simulation steps, for all human objects, and for all human sensitivities/resiliencies if a harmful event is present. For example, dedicated software components may check whether human physical integrity is compromised and dedicated statistical software components may track corresponding events.

With regard to model validation, the simulation runs may be checked against existing hazards lists for known systems or domains (sub-step 1042).

Fig. 2 illustrates a system model 20 of the technical system according to an embodiment.

The system model 20 is determined in step 101 of the above-identified computer-implemented method 10, and comprises a full physical and functional description/modeling of the technical system of interest, including models of entities/components/sub-systems to be combined according to given system requirements. The system requirements may comprise hazard-related requirements. The system entities/components/sub-systems may be modeled as having given properties, functions, functional relations and failure modes 203 with e.g. certain failure rates (see above) and/or corresponding down times and/or corresponding repair times.

As illustrated, the system model 20 may comprise at least one use case 201 of the technical system in accordance with a human interaction of the human object with the technical system. The at least one use case 201 is determined in step 102 of the above-identified computer-implemented method 10.

As illustrated, the system model 20 may further comprise a state model 2011 of the technical system in accordance with the at least one use case 201 including a plurality of operational states and at least one transition relation between the plurality of operational states. The simulating 103 of the technical system may comprise simulating 103 the technical system in at least one of the plurality of operational states of the state model 2011.

For example, the state model 2011 may be derived or defined by given system requirements and/or with the help of existing models.

As illustrated, the system model 20 of the technical system may further comprise at least one human model 202. The at least one human model 202 may comprise a model of the human interaction.

Humans may be integrated into the system model 20 as human model objects to enable reflecting human behavior and interaction as well as relevant properties such as human resilience with regard to specific loads or exposures.

The human model 202 may further comprise at least one of: a property model, a behavior model, and a resilience model of a human body.

In particular, humans may have certain properties and capabilities such as, for instance, height, size and weight, ability to hear and understand verbal messages, and having a specific reaction time. Humans may also show a certain behavior, such as, for instance, moving with a certain speed, sleeping etc., and interaction with other humans. Furthermore, humans may also possess a certain resilience or sensitivity with regard to external effects. For example, they might be hurt by high voltage or if exposed to elevated temperature over a certain time frame.

The resilience model may comprise at least one of: a whole-body damage threshold, and limb-resolved damage thresholds. The at least one threshold enables identifying 10341 that the at least one human model 202 indicates human injury.

This enables differentiating and weighting of damages affecting the whole body or individual limbs of humans.

The resilience model may comprise time-resolved damage thresholds and damage accumulation rulesets.

"Time-resolved" as used herein may refer to time variance, i.e., having different values at different time instants.

This enables taking into account time-variant sensitivities as well as nonlinear damage accumulation. For example, a damage/harm by two bleeding wounds may be set to exceed twice the damage/harm by a single bleeding wound, and high blood loss may even result in a casualty.

As illustrated, the system model 20 may further comprise at least one failure mode 203 of the technical system of: a deviation from a design or operating specification of the technical system, a random failure of a component of the technical system, a random human interaction with the component of the technical system, and a systematic human interaction with the component of the technical system. The simulating 103 of the technical system may comprise establishing 1033 the at least one failure mode 203, and in particular establishing 1033 at most one of the at least one failure mode 203 at a time.

In particular, these failure modes 203 may be defined by deviation from (predefined) functions, e.g. according to the real design if available, existing functional models including failure properties such as for instance a failure rate can be used for that purpose. Failure modes 203 may also be derived by activating functions contrary to their functional specification, e.g. untimely or with out-of-spec values, but within its physical capabilities, or following an automated Hazard and Operability Analysis, HAZOP, analysis. For example, this may imply that a door opens even though certain constraints or boundary conditions are missing, such as an "open the door signal" or despite the train is running at high speed which should be checked prior to door opening.

As illustrated, the system model 20 may further comprise an environmental model 204 of the technical system. The environmental model 204 may include at least one of: a geographical model, a climatic model, a political model, and a jurisdictional model.

The environmental model 204 may be composed by sub-models describing the landscape as well as climatic aspects. In addition, political/jurisdictional data or models are included to account for applicable local/national standards required for achieving adequate safety, e.g., the data of insurance companies about accidents or data about traffic volume or data about (critical) scenarios to be evaluated provided by e.g. the ministries of a specific country, etc. As illustrated, the system model 20 may further comprise a domain model 205 of the technical system. The domain model 205 may include at least one of: a hazard-related property, and a hazard-related rule.

For example, in a railway domain, a hazard-related property may comprise that approaching or contacting a catenary of an electrified railway line denotes a safety hazard. In particular, no specific human resiliency model is necessary to determine a hazardousness, resulting in a more effective simulation.

As a further example in the railway domain, a hazard-related rule may comprise that humans should maintain a minimum guard space/distance to the catenary. In particular, it should not be possible at all to realize an incompliant design.

The present invention may, of course, be carried out in other ways than those specifically set forth herein without departing from essential characteristics of the invention. The present embodiments are therefore to be considered in all respects as illustrative and not restrictive, and all changes coming within the meaning and equivalency range of the appended claims are intended to be embraced therein.

## Claims

1. A computer-implemented method (10) for safety analysis of a technical system comprising a human object, comprising
determining (101) a system model (20) of the technical system comprising the human object;
determining (102) at least one use case (201) of the technical system in accordance with a human interaction of the human object with the technical system; and
simulating (103) the technical system in accordance with the system model (20) and the at least one use case (201);
wherein the simulating (103) of the technical system comprises tracking (1034) of safety hazard events in relation to the human interaction.

2. The method (10) of claim 1,
wherein the system model (20) of the technical system comprises at least one human model (202); and
wherein the at least one human model (202) comprises a model of the human interaction.

3. The method (10) of claim 2,
wherein the human model (202) further comprises at least one of:
a property model,
a behavior model, and
a resilience model of a human body.

4. The method (10) of claim 3,
wherein the resilience model comprises at least one of:
a whole-body damage threshold, and
limb-resolved damage thresholds,
wherein the tracking (1034) of the safety hazard events in relation to the human interaction comprises identifying (10341) that the at least one human model (202) indicates human injury based on the at least one threshold.

5. The method (10) of claim 3 or claim 4,
wherein the resilience model of the human body comprises time-resolved damage thresholds and damage accumulation rulesets.

6. The method (10) of any one of the preceding claims,
wherein the simulating (103) of the technical system comprises establishing (1031) classes of equivalent safety hazard events with respect to the human interaction, and omitting (1032) multiple simulation runs for equivalent safety hazard events.

7. The method (10) of claim 6,
wherein the establishing (1031) of the classes of equivalent safety hazard events comprises using a method of artificial intelligence, AI.

8. The method (10) of any one of the preceding claims,
wherein the tracking (1034) of the safety hazard events in relation to the human interaction comprises at least one of: tracking (10342) a respective human injury, tracking (10343) a respective human exposure time to, and tracking (10344) a respective human avoidance behavior in relation to the tracked safety hazard events.

9. The method (10) of any one of the preceding claims, further comprising
evaluating (104) the tracked safety hazard events by evaluating a risk in accordance with at least one of: a Safety Integrity Level, SIL, evaluation model, an Automotive Safety Integrity Level, ASIL, evaluation model, a Development Assurance Level, DAL, evaluation model, a Performance Level, PL, evaluation model, and an individual evaluation model.

10. The method (10) of claim 9,
wherein the evaluating (104) of the tracked safety hazard events comprises at least one of:
statistically evaluating (1041) at least one of:
the tracked human injuries,
the tracked human exposure times, and
the tracked human avoidance behavior; and
checking (1042) the tracked safety hazard events against a list of known safety hazard events.

11. The method (10) of any one of the preceding claims,
wherein the system model (20) of the technical system further comprises at least one failure mode (203) of the technical system of:
a deviation from a design or operating specification of the technical system,
a random failure of a component of the technical system,
a random human interaction with the component of the technical system; and
a systematic human interaction with the component of the technical system; and
wherein the simulating (103) of the technical system comprises establishing (1033) the at least one failure mode (203) .

12. The method (10) of claim 11,
wherein the establishing (1033) of the at least one failure mode (203) comprises establishing at most one of the at least one failure mode (203) at a time.

13. The method (10) of any one of the preceding claims,
wherein the system model (20) of the technical system further comprises an environmental model (204) including at least one of:
a geographical model,
a climatic model,
a political model, and
a jurisdictional model.

14. The method (10) of any one of the preceding claims,
wherein the system model (20) of the technical system further comprises a domain model (205) including at least one of:
a hazard-related property, and
a hazard-related rule.

15. The method (10) of any one of the preceding claims,
wherein the system model (20) of the technical system further comprises a state model (2011) of the technical system in accordance with the at least one use case (201) including a plurality of operational states and at least one transition relation between the plurality of operational states; and
wherein the simulating (103) of the technical system comprises simulating (103) the technical system in at least one of the plurality of operational states of the state model (2011).
